# EUROPEAN PATENT APPLICATION

(11) **EP 4 458 390 A1**
(43) Date of publication of application: **06.11.2024**
(21) Application number: 23315160.4
(22) Date of filing: 04.05.2023
(51) Int. Cl.: A61M 5/315, A61M 5/31

(54) **BACKSTOP DEVICE FOR AN INJECTION DEVICE**

(71) Applicant: Becton Dickinson France, 38800 Le Pont de Claix (FR)
(72) Inventor: Jawichian, Alex, 38000 GRENOBLE (FR); Detroyat, Maël, 38120 SAINT EGREVE (FR); Perroud, Corentin, 38500 VOIRON (FR); Cado, Laurianne, 38000 GRENOBLE (FR); Chagneux, Alexandra, 38600 FONTAINE (FR)
(74) Representative: Regimbeau

(57) **Abstract**

A backstop device for maintaining a distal end of a plunger rod in a syringe barrel comprises a top plate defining a first opening configured to receive a plunger rod having an elongated body having a joining portion joining a first curved end face to a second curved end face therein. The backstop device also comprises a bottom plate defining a second opening configured to receive a syringe barrel therein and at least one connector extending between the top and bottom plates. The first opening has a shape different from a shape of the second opening. An injection device usable with the backstop device comprises a plunger rod having an elongated body with a joining portion joining first and second curved end faces that together define a discontinuous arcuate outer perimeter of the elongated body.

## Description

### TECHNICAL FIELD

The disclosed concept relates generally to a backstop device usable with an injection device such that the backstop device maintains a distal end of a plunger rod and, more particularly, a stopper on the distal end of the plunger rod within a syringe barrel.

### BACKGROUND

Syringes are medical delivery devices configured to administer a medicinal fluid (e.g., pharmaceutical, medicament) to a patient. Syringes may be sold in a prefilled form, wherein medicinal fluid is disposed within the syringe at the time of manufacture of the syringe, or in an empty form, wherein medicinal fluid is filled from a vial or other source by an end user close in time to administering the medicinal fluid with the syringe.

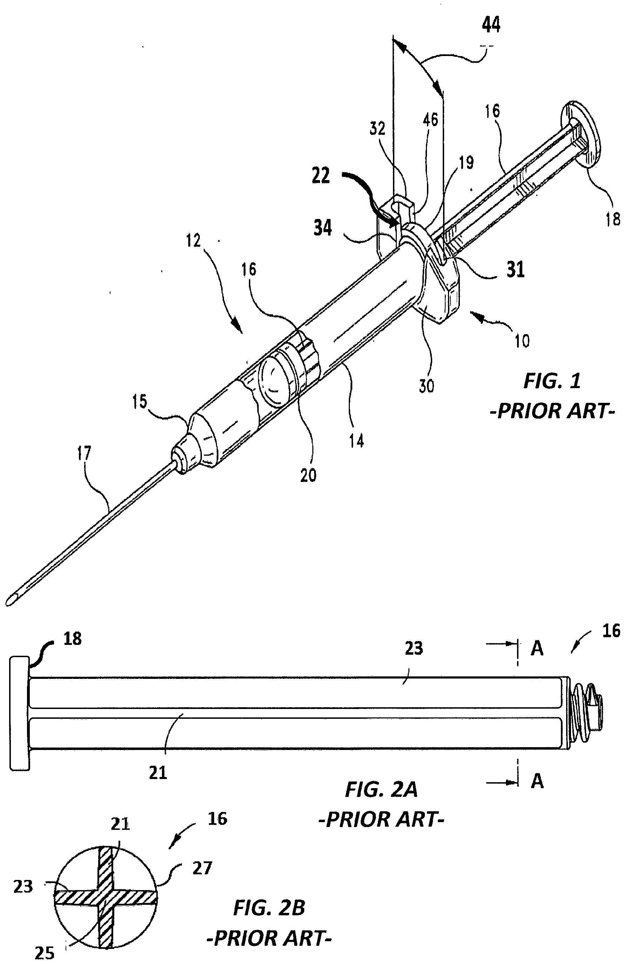

FIGS. 1, 2A, and 2B illustrate various parts of an injection device 12 including a syringe barrel 14 on which a backstop 10 is provided and in which a plunger rod 16 is disposed. An example of an injection device including a syringe barrel, plunger rod, and backstop is provided in US Patent No. 5,667,495.

With continued reference to FIGS. 1, 2A, and 2B, the injection device 12 generally includes a syringe barrel 14 configured to contain a medicament therein, with the barrel 14 having a distal end 15 to which a piercing element 17 (e.g., a needle) is coupled and having an open proximal end 19 at which a flange 22 is located.

The plunger rod 16 is disposed in the proximal end 19. As best illustrated in the cross-sectional view of FIG. 2B, which is taken in the plane A as indicated in FIG. 2A, the plunger rod 16 of the prior art includes a first planar portion 21 and a second planar portion 23 that intersects first planar portion 21 at a central region 25. In general, the plunger rod 16 has a cross-shaped cross-section when viewed in plane A. The plunger rod 16 has a finger rest 18 at a proximal end thereof and a stopper 20 at a distal end thereof.

The backstop 10 is configured to be mounted on and used in cooperation with the plunger rod 16. The backstop 10 of the prior art includes a bottom plate 30 and a top plate 32. The plates 30, 32 define an enclosure 31 therebetween. The enclosure 31 is further defined by a sidewall extending generally vertically between the bottom and top plates 30, 32 and along three sides of the bottom and top plates 30, 32. The three-sided sidewall extends about a majority of the periphery of the backstop 10 and substantially encloses the flange 22 of syringe barrel 14 therein. A frontal opening 44 is provided to allow for insertion of the flange 22 therein.

An aperture is provided in each of the plates 30, 32. The aperture 34 of the bottom plate 30 is configured and dimensioned for insertion of and form fitting contact with a peripheral surface of the syringe barrel 14. The aperture 34 of the bottom plate 30 includes a portion formed in a relatively arcuate manner so as to conform to the generally cylindrical exterior surface of the syringe barrel 14. More particularly, the aperture 34 is defined by a curved edge of the bottom plate 30. A lead opening of the aperture 34 extends from the curved edge and is defined by two diverging and generally planar surfaces. The lead opening of the aperture 34 is generally formed in a manner to accommodate insertion of the syringe barrel 14 therethrough.

The aperture 46 of the top plate 32 is configured and dimensioned to accommodate insertion of the plunger rod 16 therein. The aperture 46 of the top plate 32 is formed in a substantially similar manner to the aperture 34 of the bottom plate 30. In particular, the aperture 46 of the top plate 32 includes a portion formed in a relatively arcuate manner by a curved edge so as to conform to the shape of a circle 27 which inscribes the discontinuous arcuate outer perimeter of the plunger rod 16 as defined by the exterior surfaces of the intersecting planar portions 21, 23. A lead opening extends from the curved edged edge and is defined by two diverging and generally planar surface. The lead opening of the aperture 46 is generally formed in a manner to accommodate insertion of the plunger rod 16 therethrough.

In use, the plunger rod 16 may be advanced toward the distal end 15 of the barrel 14 in order to expel a fluid, such as a medicament, therefrom. The plunger rod 16 may also be retracted from the distal end 15 and toward the proximal end 19 to aspirate or fill the syringe barrel 14 with a fluid. The backstop 10 is configured to permit movement of the plunger rod 16 in each direction and to prevent the plunger rod 16 from being removed from the barrel 14.

Numerous syringes are used each year in medical settings. As a result, it is desirable that syringes be manufactured as cost efficiently as possible. To this end, plunger rod designs are being modified to reduce material usage and manufacturing time. Modifications to the plunger rod permit modifications to the associated backstop to be made accordingly and permit the backstop to be modified to reduce material usage and manufacturing time.

### BRIEF SUMMARY

An objective of the present disclosure is to provide a backstop device for cooperation with an injection device including a plunger rod solving the above-mentioned drawbacks.

A backstop device is for maintaining a distal end of a plunger rod in a syringe barrel. The backstop device comprises a top plate defining a first opening that is configured to receive a plunger rod having an elongated body having a joining portion joining a first curved end face to a second curved end face therein. The backstop device also comprises a bottom plate defining a second opening that is configured to receive a syringe barrel therein. The first opening has a shape different from a shape of the second opening. The backstop device further comprises at least one connector extending between the top plate and the bottom plate.

Certain preferred but non-limiting features of the backstop device described above are the following, taken individually or in combination:
the first opening of the top plate is defined by a first planar surface, a second planar surface, and a third planar surface, the first planar surface and the second planar surface being located proximate to opposite ends of the third planar surface and extending away from the third planar surface in a parallel manner or in a diverging manner;
a maximum depth of the top plate is less than a maximum depth of the bottom plate;
a maximum width of the top plate is less than a maximum width of the bottom plate;
the second opening of the bottom plate is at least partially defined by an arcuate surface, a curvature of the arcuate surface corresponding to a curvature of an exterior surface of the syringe barrel receivable therein;
an upper surface and a lower surface of the top plate extend substantially parallel to each other;
the at least one connector of the backstop device extends along a portion of a first side surface of the top plate and along a portion of a first side surface of the bottom plate, the first side surface of the top plate being opposite a second side surface of the top plate in which the first opening is open such that the plunger rod is receivable therein, the first side surface of the bottom plate being opposite a second side surface of the bottom plate in which the second opening is open such that the syringe barrel is receivable therein;
the backstop device comprises two opposing connectors, each of the two connectors extending from a first side surface of the top plate and a first side surface of the bottom plate toward a second side surface of the top plate and a second side surface of the bottom plate; and/or
a volume of the top plate is less than a volume of the bottom plate.

An injection device comprises a syringe barrel, a plunger rod, and the backstop device as described above. The plunger rod comprises an elongated body extending along a central axis between a proximal end and a distal end. The body has a joining portion joining a first curved end face to a second curved end face. Each of the first curved end face, the second curved end face, and the joining portion extends between the proximal end and the distal end, wherein the first curved end face and the second curved end face together define a discontinuous arcuate outer perimeter of the elongated body.

Certain preferred but non-limiting features of the injection device described above are the following, taken individually or in combination:
a distance between a first planar surface and a second planar surface of the first opening of the top plate is less than a diameter of the distal end of the plunger rod;
the bottom plate is separated from the top plate by a distance such that, when the backstop device is coupled to the syringe barrel, a lower surface of the top plate and an upper surface of the bottom plate contact an upper surface and a lower surface of a flange of the syringe barrel, respectively.

### BRIEF DESCRIPTION OF THE DRAWINGS

The illustrations presented herein are not meant to be actual views of any particular component, device, or system, but are merely idealized representations which are employed to describe embodiments of the present invention. Other features, goals, and advantages of the present invention will appear more clearly upon reading of the detailed description that follows and with references to drawings provided by way of non-limiting examples wherein:
FIG. 1 is a perspective view of an injection device according to the prior art;
FIGS. 2A and 2B illustrate a plunger rod of the injection device of FIG. 1 in a side view and cross-sectional view, respectively;
FIG. 3 is a perspective view of a plunger rod;
FIG. 4 is a side view of the plunger rod disposed in a syringe barrel;
FIG. 5 is a cross-sectional view of the plunger rod of FIG. 3;
FIGS. 6-8 illustrate a backstop in a cross-sectional view, a perspective view, and a top view, respectively;
FIG. 9 illustrates a further backstop in a perspective view;
FIGS. 10 and 11 illustrate another backstop in a top view and perspective view, respectively, and FIG. 12 illustrates a modification of the backstop of FIGS. 10 and 11 in perspective view; and
FIGS. 13 and 14 illustrates yet another backstop in a perspective view and top view, respectively, and FIG. 15 illustrates a modification of the backstop of FIGS. 13 and 14 in perspective view.

### DETAILED DESCRIPTION

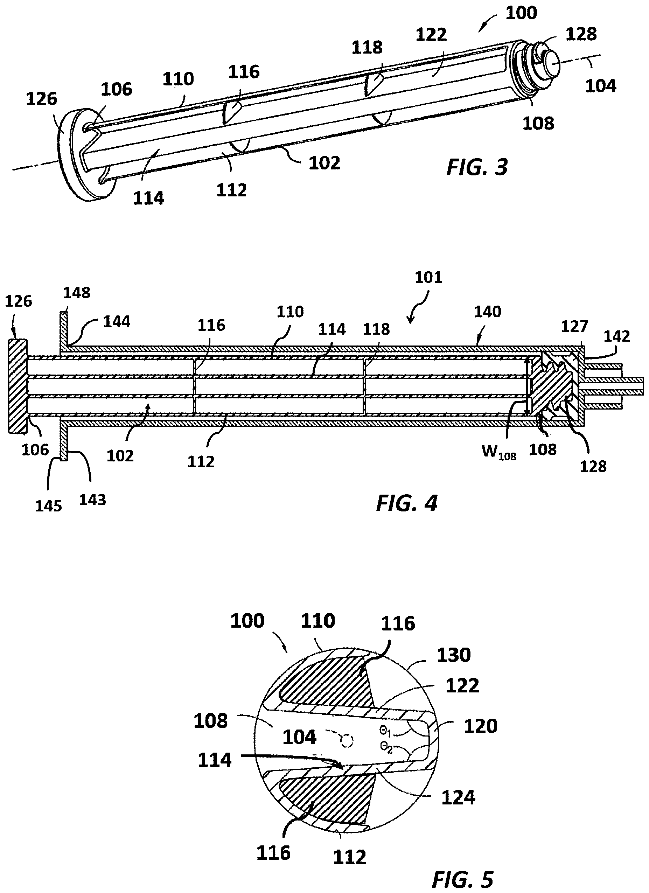

FIG. 3 is a perspective view of a plunger rod 100. FIG. 4 is a side view of the plunger rod 100 disposed in a syringe barrel 140. FIG. 5 is a cross-sectional view of the plunger rod 100.

The plunger rod 100 includes an elongated body 102 having a central axis 104 extending between a proximal end 106 and a distal end 108. In one embodiment, the central axis 104 of the plunger rod 100 may coincide with a corresponding central axis of the syringe barrel 140. The elongated body 102 of plunger rod 100 also includes a first curved end face 110, a second curved end face 112, and a joining portion 114 which joins the first curved end face 110 to the second curved end face 112. The first and second curved end faces 110 and 112 may also be formed of straight line segments or a plurality of corresponding adjacent segments. In one example embodiment, each of the first curved end face 110, the second curved end face 112, and the joining portion 114 extends between the proximal end 106 and the distal end 108. At least the first curved end face 110 and the second curved end face 112 together may be inscribed by the circle defined by an outer perimeter 130 (shown in FIG. 5) of elongated body 102. As shown, the outer perimeter 130 may be discontinuous. As will be discussed below, the geometry of plunger rod 100 allows plunger rod 100 to be manufactured with less material and molded with a shorter cycle time.

The plunger rod 100 may optionally further include a thumb press 126 located at the proximal end 106 of the elongated body 102 and a threaded coupling portion 128 located at the distal end 108 of the elongated body 102. The thumb press 126 may include a portion that is wider than an interior dimension of the syringe barrel 140 and provides a structure for a user to press when advancing the plunger rod 100 through the syringe barrel 140. The circumference and diameter of the arcuate outer perimeter 130 may be less than the circumference and diameter of an arcuate outer perimeter of the thumb press 126.

A stopper 127 may be threadably coupled to the coupling portion 128. Alternatively, the stopper 127 may be clipped onto plunger rod 100. In operation, a user may apply a force on the thumb press 126 and advance the plunger rod 100 and stopper 127 within the syringe barrel 140. The stopper 127 is sized relative to syringe barrel 140 to provide sealing engagement with the interior surface of the sidewall of syringe barrel 140. It is noted herein that any suitable stopper may be employed with the plunger rod 100.

When assembled, the elongated body 102 is at least partially disposed within the syringe barrel 140. The syringe barrel 140 may be in the general form of an elongated cylindrical barrel, though the syringe barrel 140 may be in other forms for containing a fluid for delivery.

The syringe barrel 140 extends axially between a distal end 142 and a proximal end 144. The barrel 140 is configured to contain a medical fluid therein. The barrel 140 comprises an inner surface defining a hollow space in which the stopper 127 may be disposed. A cannula (e.g., needle) may be coupled to the distal end 142 for injecting the medicinal fluid to a patient. The needle may be stacked on the distal end 142 or may be coupled, such as by threading, to the distal end 142 via an adaptor, which may be snap-fitted or glued to the distal end 142.

The syringe barrel 140 may be formed of glass, or may be injection molded from thermoplastic material such as polypropylene and polyethylene according to techniques known to those of ordinary skill in the art, though it is to be appreciated that syringe barrel 140 may be made from other suitable materials and according to other applicable techniques.

With continued reference to FIGS. 3 and 5, the plunger rod 100 may comprise a plurality of support ribs (two support ribs 116, 118 are shown) extending from elongated body 102 between proximal end 106 and distal end 108. The support ribs 116 and 118 may extend radially, such as orthogonally, with respect to central axis 104, and a plunger rod in accordance with the disclosed concept may include up to five ribs.

As best illustrated in FIG. 5, the joining portion 114 includes an intermediate face 120, a first connecting face (e.g., without limitation, first angular face 122) extending from intermediate face 120 to the first curved end face 110, and a second connecting face (e.g., without limitation, second angular face 124) extending from the intermediate face 120 to the second curved end face 112.

The angular faces 122 and 124 may be curved or straight. The joining portion 114 at least partially defines the arcuate outer perimeter 130 of the elongated body 102, which may be concave facing toward central axis 104. In one example embodiment, the intermediate face 120 and the curved end faces 110, 112 may be inscribed by the circle delimited by the outer perimeter 130 of the elongated body 102 together therewith. Additionally, the intermediate face 120 may be curved or straight. Accordingly, it will be appreciated that the arcuate outer perimeter 130 may be discontinuous.

Furthermore, the first and second connecting faces may include the first and second angular faces 122, 124, respectively, with respect to the intermediate face 120. More specifically, in one example embodiment, the first and second angular faces 122, 124 each extend from the intermediate face 120 at respective angles θ₁, θ₂ that are equal to or greater than 90 degrees with respect to the intermediate face 120. Preferably the angles θ₁, θ₂ are smaller than 120 degrees with respect to the intermediate face 120. Additionally, in one configuration, no portion of the first curved end face 110, the second curved end face 112, and/or the joining portion 114 intersects the central axis 104 (depicted as a circle in order to represent an axis perpendicular with the page) of elongated body 102. Accordingly, in this configuration, the central axis 104 of the plunger rod 100 is void of material. It is further noted that in certain embodiments, the first curved end face 110 and the second curved end face 112 do not intersect each other, i.e., the first curved end face 110 and the second curved end face 112 do not form a continuous exterior perimeter.

In operation, the geometry of plunger rod 100 provides a number of advantages over known plunger rods such as the plunger rod 16 shown in FIGS. 2A and 2B. For example, by having a discontinuous arcuate outer perimeter 130, and having sections that do not intersect each other, elongated body 102 can advantageously be manufactured using less material than a conventional plunger rod 100. As previously discussed with reference to FIG. 2B, the first planar portion 21 and the second planar portion 23 intersect at the central region 25. It can readily be appreciated that central region 25 has an undesirably large amount of material due to the intersection of the first and second planar portions 21, 23. This is disadvantageous at least for the following reasons. First, by having a large amount of material in this region, material costs are relatively high to manufacture plunger rod 16 due to the increased amount of material present within this region. Second, cycle times associated with molding plunger rod 16 are relatively long, due to the time required for the large central region 25 to cool.

The plunger rod 100 of FIGS. 3-5 solves these problems by having a void at the central axis 104 which center area might otherwise generate large masses of material and associated costs can be reduced. In addition, the plunger rod 100 is able to resist breakage with better resistance to flexion (e.g., displacement away from central axis 104) during movement within the syringe barrel 140) than conventional plunger rod 16, and are able to do so using significantly less material. Due to the increased resistance to breakage, plunger rods 100, unlike plunger rod 16, may be manufactured out of different materials (e.g., without limitation, thermoplastic material such as a polycarbonate material, polypropylene, polystyrene, etc.).

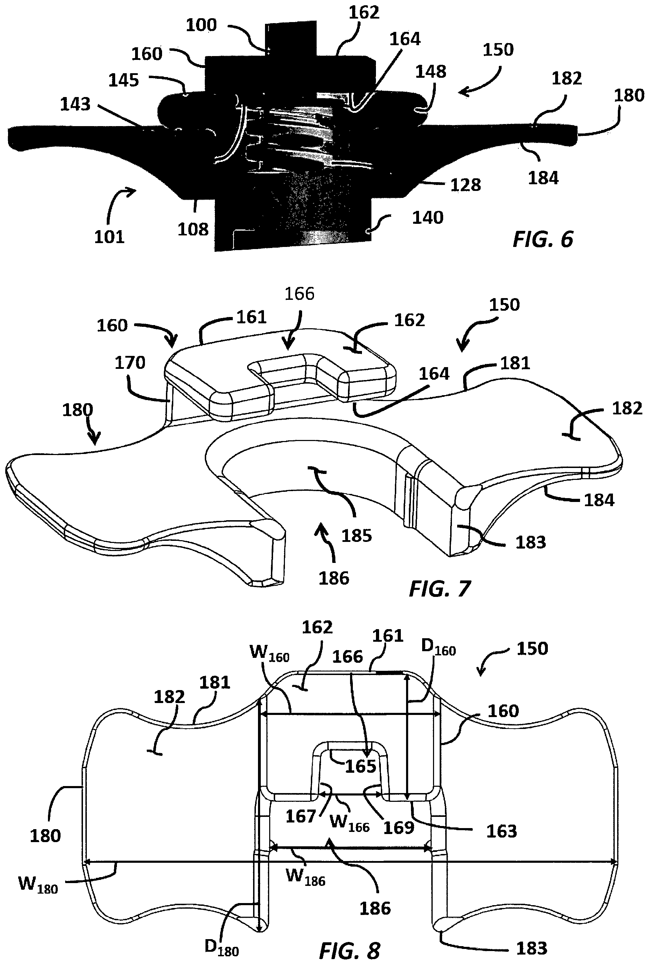

FIGS. 6-9 illustrate a backstop 150 useable with the plunger rod 100 as described herein. The backstop 150 includes a top plate 160 and a bottom plate 180. At least one connector 170 extends between and connects the top plate 160 and the bottom plate 180.

The top plate 160 has an upper surface 162 and an opposite lower surface 164. In some embodiments, the upper surface 162 and the lower surface 164 may be substantially planar and may extend parallel to each other.

The bottom plate 180 has an upper surface 182 and an opposite lower surface 184. The bottom plate 180 is designed such that a user may grip the injection device 101 and displace the plunger rod 100 within the barrel 140. The lower surface 184 of the bottom plate 180 may comprise one or more concave portions shaped for receiving an index and/or middle finger of the user to grip the injection device 101 while a thumb of the user is placed on the thumb press 126 of the plunger rod 100.

The lower surface 164 and the upper surface 182 of the bottom plate 180 are separated by a distance and define a space therebetween. The space is sized to retain the flange 148 of the syringe barrel 140 therein. For instance, a height of the space may be substantially equal to a thickness of the flange 148 measured between the upper surface 145 and a lower surface 143 of the flange 148.

The top plate 160 includes a first opening 166. The first opening 166 is configured to receive the plunger rod 100 therein and allow the elongated body 102 of the plunger rod 100 to pass therethrough. The first opening 166 extends fully through the top plate 160 (e.g., from the upper surface 162 to the lower surface 164).

At least three planar surfaces 165, 167, 169 define the first opening 166. First and second planar surfaces 167, 169 are located opposite each other and proximate to opposite ends of the third planar surface 165. The first and second planar surfaces 167, 169 may extend parallel to each other as the planar surfaces 167, 169 extend away from the third planar surface 165. The first and second planar surfaces 167, 169 may extend in a diverging manner as the planar surfaces 167, 169 extend away from the third planar surface 165.

The first and second planar surfaces 167, 169 may extend substantially parallel to the angular faces 122, 124 of the plunger rod 100. Accordingly, the plunger rod 100 may pass through the first opening 166 as the plunger rod 100 is advanced within the syringe barrel 140 when the user applies a force on the thumb press 126.

The bottom plate 180 includes a second opening 186. The second opening 186 has a size and shape different from the first opening 166. The second opening 186 is configured to retain the syringe barrel 140 therein. The surface 185 defining the second opening is generally arcuate to correspond to the arcuate outer surface of the syringe barrel 140.

In one arrangement, as illustrated in FIGS. 6 and 7, the backstop 150 comprises one connector 170 extending along a portion of a first side surface 161 of the top plate 160 and along a portion of a first side surface 181 of the bottom plate 180. The first side surface 161 of the top plate 160 is located opposite a second side surface 163 of the top plate 160 in which the first opening 166 is open such that the plunger rod 110 is receivable therein during assembly of the injection device. The first side surface 181 of the bottom plate 180 is located opposite a second side surface 183 of the bottom plate 180 in which the second opening 186 is open such that the syringe barrel 101 is receivable therein.

The connector 170 extends generally vertically between the top and bottom plates 160, 180 and along one side of the top and bottom plates 160, 180 such that a majority of the periphery of the flange 148 of the syringe barrel 140 is unbounded by the backstop 150.

In another arrangement, as illustrated in FIG. 8, the backstop 150 comprises two opposing connectors 170. Each of the two connectors 170 extends from the first side surface 161 of the top plate 160 and the first side surface 181 of the bottom plate 180 toward the second side surface 163 of the top plate 160 and the second side surface 183 of the bottom plate 180.

The provision of two opposing connectors 170 results in an increase in the width W₁₆₀ of the top plate 160 while maintaining the depth D₁₆₀ relative to the top plate 160 having a single connector 170. The two opposing connectors 170 extend along two sides of the top and bottom plates 160, 180 such that a majority of the periphery of the flange 148 of the syringe barrel 140 is unbounded by the backstop 150.

In either embodiment, the connector(s) 170 extend(s) about a minority of the periphery of the space in which the flange 148 of the syringe barrel 140 is received. This limits the cost and time of manufacturing by reducing material usage.

The top plate 160 is designed to prevent the stopper 127 coupled to the coupling portion 128 of the plunger rod 100 from being removed from the syringe barrel 140 while allowing the elongated body 102 to pass through the first opening 126 when the plunger rod 100, the syringe barrel 140, and the backstop 150 are assembled together. The top plate 160 has a width W₁₆₀ and a depth D₁₆₀ sufficient to provide an abutment for the upper surface 145 of the flange 148.

The top plate 160 is dimensioned so as to minimize the amount of material used in its construction. The width W₁₆₀ of the top plate 160 is less than a width W₁₈₀ of the bottom plate 180. The depth D₁₆₀ of the top plate 160 is less than a depth Disc of the bottom plate 180. Thus, a volume of the top plate 160 is less than a volume of the bottom plate 180.

The depth D₁₆₀ of the top plate 160 is selected to avoid contact between the support ribs 116, 118 and the top plate 160 such that the plunger rod 100 passes through the first opening 166 when the user applies a force on the thumb press 126.

A width W₁₆₆ of the first opening 166 of the top plate 160 may be less than a width Wise of the second opening 186 of the bottom plate 180. The width W₁₆₆ of the first opening 166 is selected to be less than a width W₁₀₈ of the distal end 108 of the plunger rod 100 to prevent withdrawal of the distal end 108 of the plunger rod 100 from the syringe barrel 140. The W₁₆₆ is selected to be greater than the distance between the angular faces 122, 124 of the joining portion 114 such that the plunger rod 100 passes through the first opening 166 when the user applies a force on the thumb press 126.

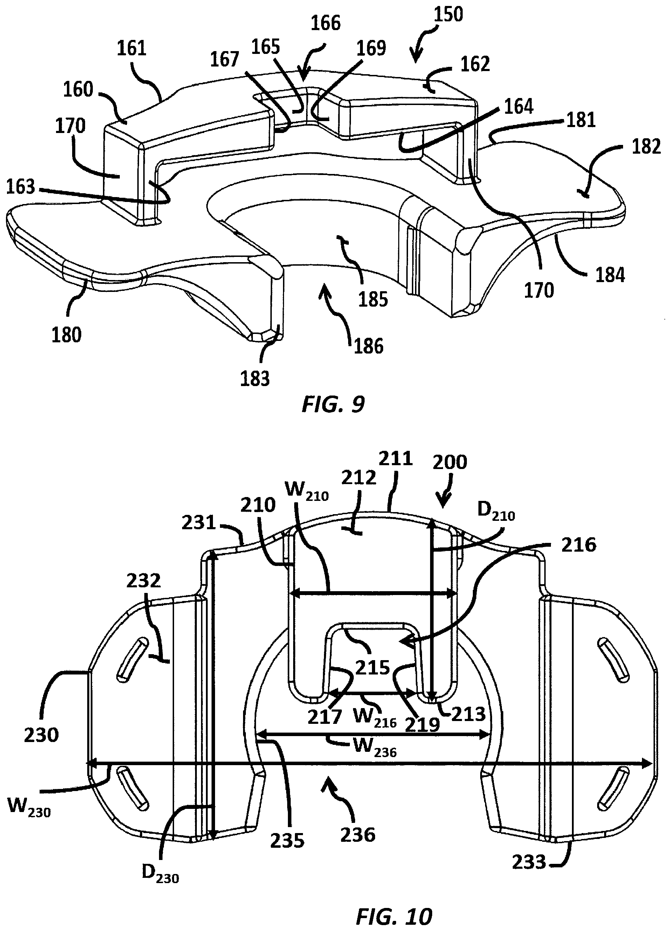

FIGS. 10-12 illustrate another backstop 200 useable with the plunger rod 100 as described herein. The backstop 200 includes a top plate 210, a bottom plate 230, and at least one connector 220 extending therebetween. The backstop 200 is substantially similar to the backstop 160 except that the overall shape of the bottom plate 230 and the top plate 210 are different from the top plate 160 and bottom plate 170.

Like the top plate 160, the top plate 210 has an upper surface 212 and an opposite lower surface 214, which may be substantially planar and may extend parallel to each other.

The bottom plate 230 has an upper surface 212 and an opposite lower surface 214. The bottom plate 230 is designed such that a user may grip the injection device 101 and displace the plunger rod 100 within the barrel 140. The lower surface 234 of the bottom plate 230 is generally planar and is configured for an index and/or middle finger of the user to rest therein in order to grip the injection device 101 while a thumb of the user is placed on the thumb press 126 of the plunger rod 100.

The lower surface 234 and the upper surface 232 of the bottom plate 230 are separated by a distance and define a space therebetween, which space is sized to retain the flange 148 of the syringe barrel 140 therein as previously described.

The top plate 210 includes a first opening 216. Like the top plate 160, at least three planar surfaces 215, 217, 219 define the first opening 166. The first and second planar surfaces 215, 217 may extend parallel to each other or in a diverging manner as the planar surfaces 217, 219 extend away from the third planar surface 165.

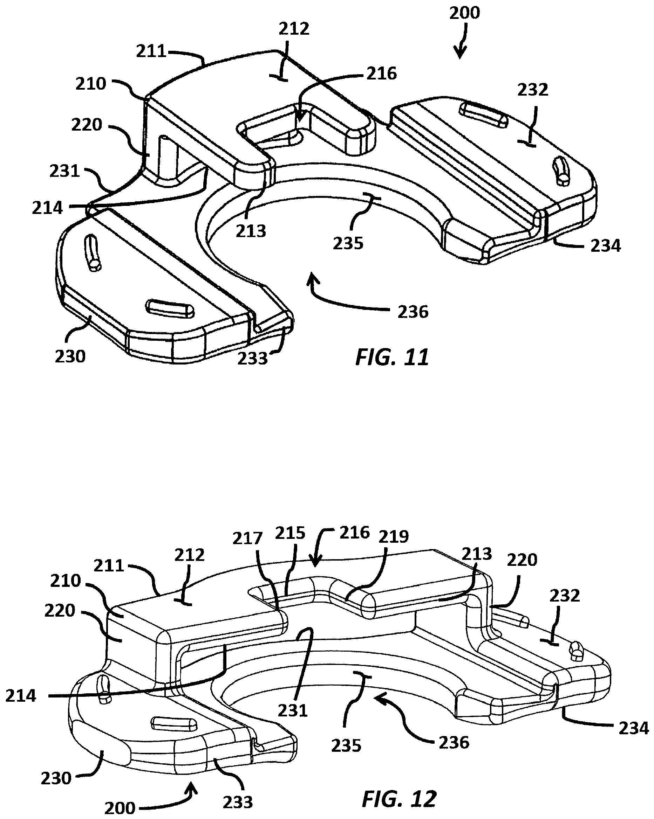

The bottom plate 230 includes a second opening 236, which is configured to retain the syringe barrel 140 therein. In one arrangement, as illustrated in FIGS. 10 and 11, the backstop 200 comprises one connector 220 extending along a portion of a first side surface 211 of the top plate 210 and along a portion of a first side surface 231 of the bottom plate 230. The first side surface 211 of the top plate 210 being opposite a second side surface 213 of the top plate 210 in which the first opening 216 is open such that the plunger rod 110 is receivable therein during assembly of the injection device. The first side surface 231 of the bottom plate 230 being opposite a second side surface 233 of the bottom plate 230 in which the second opening 236 is open such that the syringe barrel 101 is receivable therein.

In another arrangement, as illustrated in FIG. 12, the backstop 200 comprises two opposing connectors 220. Each of the two connectors 220 extends from the first side surface 211 of the top plate 210 and the first side surface 231 of the bottom plate 230 toward the second side surface 213 of the top plate 210 and the second side surface 233 of the bottom plate 230.

In either embodiment, the connector(s) 220 extend(s) generally vertically between the top and bottom plates 210, 230 and along one or two sides of the top and bottom plates 210, 230 such that a majority of the periphery of the flange 148 of the syringe barrel 140 is unbounded by the backstop 200. This limits the cost and time of manufacturing by reducing material usage.

The provision of two opposing connectors 220 results in an increase in the width W₂₁₀ of the top plate 210 while maintaining the depth D₂₁₀ relative to the top plate 210 having a single connector 220. The two opposing connectors 220 extend along two sides of the top and bottom plates 210, 230 such that a majority of the periphery of the flange 148 of the syringe barrel 140 is unbounded by the backstop 200.

A size and shape of the first opening 216 of the top plate 210 is different from a shape of the second opening 236 of the bottom plate 230. As was explained with reference to the backstop 150, the first opening 216 is configured to receive the plunger rod 100 therein and allow the elongated body 102 of the plunger rod 100 to pass therethrough. The first opening 216 extends fully through the top plate 210 (e.g., from the upper surface 212 to the lower surface 214).

The top plate 210 is designed to prevent the stopper 127 coupled to the coupling portion 128 of the plunger rod 100 from being removed from the syringe barrel 140 when the plunger rod 100, the syringe barrel 140, and the backstop 200 are assembled together. The top plate 210 has a width W₂₁₀ and a depth D₂₁₀ sufficient to provide an abutment for the upper surface 145 of the flange 148 so as to maintain the syringe barrel 140 within the backstop 200.

The top plate 210 is dimensioned so as to minimize the amount of material used in its construction. The width W₂₁₀ of the top plate 210 is less than a width W₂₃₀ of the bottom plate 230. Further, the depth D₂₁₀ of the top plate 210 is less than a depth D₂₃₀ of the bottom plate 230. Thus, a volume of the top plate 210 is less than a volume of the bottom plate 230.

The depth D₂₁₀ of the top plate 210 is selected to avoid contact between the support ribs 116, 118 and the top plate 210 such that the plunger rod 100 passes through the first opening 216 when the user applies a force on the thumb press 126.

A width W₂₁₆ of the first opening 216 of the top plate 210 may be less than a width W₂₃₆ of the second opening 236 of the bottom plate 230. The width W₂₁₆ of the first opening 216 is selected to be less than a width W₁₀₈ of the distal end 108 of the plunger rod 100 to prevent withdrawal of the distal end 108 of the plunger rod 100 from the syringe barrel 140. The W₂₁₆ is selected to be greater than the distance between the angular faces 122, 124 of the joining portion 114 such that the plunger rod 100 passes through the first opening 216 when the user applies a force on the thumb press 126.

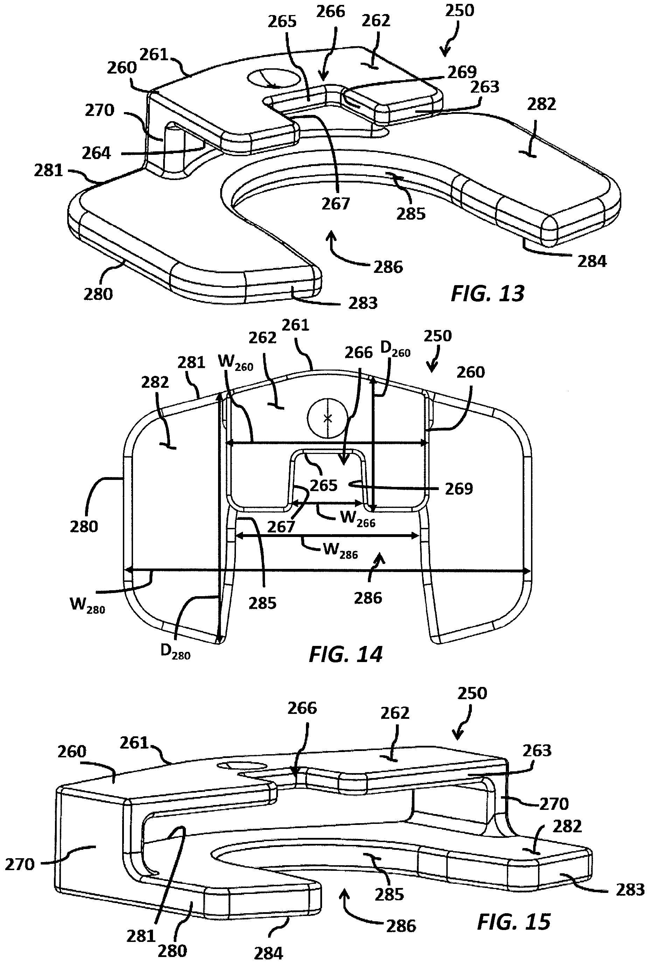

FIGS. 13-15 illustrate a further embodiment of a backstop 250 useable with the plunger rod 100 as described herein. The backstop 250 includes a top plate 260, a bottom plate 280, and at least one connector 270 extending therebetween. The backstop 250 is substantially similar to the backstop 160 except that the overall shape of the bottom plate 280 and the top plate 260 are different from the top plate 160 and bottom plate 170.

Like the top plate 160, the top plate 260 has an upper surface 262 and an opposite lower surface 264, which may be substantially planar and may extend parallel to each other.

The bottom plate 280 has an upper surface 282 and an opposite lower surface 284. The bottom plate 280 is designed such that a user may grip the injection device 101 and displace the plunger rod 100 within the barrel 140. The lower surface 284 of the bottom plate 280 is generally planar and is configured for an index and/or middle finger of the user to rest therein in order to grip the injection device 101 while a thumb of the user is placed on the thumb press 126 of the plunger rod 100.

The lower surface 284 and the upper surface 282 of the bottom plate 280 are separated by a distance and define a space therebetween, which space is sized to retain the flange 148 of the syringe barrel 140 therein as previously described.

The top plate 260 includes a first opening 266. Like the top plate 160, at least three planar surfaces 265, 267, 269 define the first opening 266. The first and second planar surfaces 265, 267 may extend parallel to each other or in a diverging manner as the planar surfaces 267, 269 extend away from the third planar surface 265.

The bottom plate 280 includes a second opening 286, which is configured to retain the syringe barrel 140 therein. In one arrangement, as illustrated in FIGS. 10 and 11, the backstop 250 comprises one connector 270 extending along a portion of a first side surface 261 of the top plate 260 and along a portion of a first side surface 281 of the bottom plate 280. The first side surface 261 of the top plate 260 being opposite a second side surface 263 of the top plate 260 in which the first opening 266 is open such that the plunger rod 110 is receivable therein during assembly of the injection device ###. The first side surface 281 of the bottom plate 280 being opposite a second side surface 283 of the bottom plate 280 in which the second opening 286 is open such that the syringe barrel 101 is receivable therein.

In another arrangement, as illustrated in FIG. 12, the backstop 250 comprises two opposing connectors 270. Each of the two connectors 270 extends from the first side surface 261 of the top plate 260 and the first side surface 281 of the bottom plate 280 toward the second side surface 263 of the top plate 260 and the second side surface 283 of the bottom plate 280.

The provision of two opposing connectors 270 results in an increase in the width W₂₆₀ of the top plate 260 while maintaining the depth D₂₆₀ relative to the top plate 260 having a single connector 270. The two opposing connectors 270 extend along two sides of the top and bottom plates 260, 280 such that a majority of the periphery of the flange 148 of the syringe barrel 140 is unbounded by the backstop 250.

In either embodiment, the connector(s) 270 extend(s) generally vertically between the top and bottom plates 260, 280 and along one or two sides of the top and bottom plates 260, 280 such that a majority of the periphery of the flange 148 of the syringe barrel 140 is unbounded by the backstop 250. This limits the cost and time of manufacturing by reducing material usage.

A size and shape of the first opening 266 of the top plate 260 is different from a shape of the second opening 286 of the bottom plate 280. The first opening 266 is configured to receive the plunger rod 100 therein and allow the elongated body 102 of the plunger rod 100 to pass therethrough. The first opening 266 extends fully through the top plate 260 (e.g., from the upper surface 262 to the lower surface 264).

The top plate 260 is designed to prevent the stopper 127 coupled to the coupling portion 128 of the plunger rod 100 from being removed from the syringe barrel 140 when the plunger rod 100, the syringe barrel 140, and the backstop 250 are assembled together. The top plate 260 has a width W₂₆₀ and a depth D₂₆₀ sufficient to provide an abutment for the upper surface 145 of the flange 148.

The top plate 260 is dimensioned so as to minimize the amount of material used in its construction. The width W₂₆₀ of the top plate 260 is less than a width W₂₈₀ of the bottom plate 280. The depth D₂₆₀ of the top plate 260 is less than a depth D₂₈₀ of the bottom plate 280. Thus, a volume of the top plate 260 is less than a volume of the bottom plate 280.

A width W₂₆₆ of the first opening 266 of the top plate 260 may be less than a width W₂₈₆ of the second opening 286 of the bottom plate 280. The width W₂₆₆ of the first opening 266 is selected to be less than a width W₁₀₈ of the distal end 108 of the plunger rod 100 to prevent withdrawal of the distal end 108 of the plunger rod 100 from the syringe barrel 140. The W₂₆₆ is selected to be greater than the distance between the angular faces 122, 124 of the joining portion 114 such that the plunger rod 100 passes through the first opening 266 when the user applies a force on the thumb press 126.

## Claims

1. A backstop device (150, 200, 250) for maintaining a distal end (108) of a plunger rod (110) in a syringe barrel (140), the backstop device (150, 200, 250) comprising:
a top plate (160, 210, 260) defining a first opening (166, 216, 266) that is configured to receive a plunger rod (110) having an elongated body (102) having a joining portion (114) joining a first curved end face (110) to a second curved end face (112) therein;
a bottom plate (180, 230, 280) defining a second opening (186, 236, 286) that is configured to receive the syringe barrel (140) therein, wherein the first opening (166, 216, 266) has a shape different from a shape of the second opening (186, 236, 286); and
at least one connector (170, 220, 270) extending between the top plate (160, 210, 260) and the bottom plate (180, 230, 280).

2. The backstop device (150, 200, 250) of claim 1, wherein the first opening (166, 216, 266) of the top plate (160, 210, 260) is defined by a first planar surface (167, 217, 267), a second planar surface (169, 219, 269), and a third planar surface (165, 215, 265), the first planar surface and second planar surface being located proximate to opposite ends of the third planar surface (165) and extending away from the third planar surface in a parallel manner or in a diverging manner.

3. The backstop device (150, 200, 250) of any of claims 1-2, wherein a maximum depth (D₁₆₀, D₂₁₀, D₂₆₀) of the top plate (160, 210, 260) is less than a maximum depth (D₁₈₀, D₂₃₀, D₂₈₀) of the bottom plate (180, 230, 280).

4. The backstop device (150, 200, 250) of any of claims 1-3, wherein a maximum width (W₁₆₀, W₂₁₀, W₂₆₀) of the top plate (160, 210, 260) is less than a maximum width (W₁₈₀, W₂₃₀, W₂₈₀) of the bottom plate (180, 230, 280).

5. The backstop device (150, 200, 250) of any of claims 1-4, wherein the second opening (186, 236, 286) of the bottom plate (180, 230, 280) is at least partially defined by an arcuate surface (185, 235, 285), a curvature of the arcuate surface (185, 235, 285) corresponding to a curvature of an exterior surface of the syringe barrel (140) receivable therein.

6. The backstop device (150, 200, 250) of any of claims 1-5, wherein an upper surface (162, 212, 262) and a lower surface (164, 214, 264) of the top plate (160, 210, 260) extend substantially parallel to each other.

7. The backstop device (150, 200, 250) of any of claims 1-6, wherein the at least one connector (170, 220, 270) of the backstop device (150, 200, 250) extends along a portion of a first side surface (161, 211, 261) of the top plate (160, 210, 260) and along a portion of a first side surface (181, 231, 281) of the bottom plate (180, 230, 280), the first side surface (161, 211, 261) of the top plate (160, 210, 260) being opposite a second side surface (163, 213, 263) of the top plate (160, 210, 260) in which the first opening (166, 216, 266) is open such that the plunger rod (110) is receivable therein, the first side surface (181, 231, 281) of the bottom plate (180, 230, 280) being opposite a second side surface (183, 233, 283) of the bottom plate (180, 230, 280) in which the second opening (186, 236, 286) is open such that the syringe barrel (140) is receivable therein.

8. The backstop device (150, 200, 250) of any of claims 1-6, wherein the at least one connector (170, 220, 270) of the backstop device (150, 200, 250) comprises two opposing connectors (170, 220, 270), each of the two connectors (170, 220, 270) extending from a first side surface (161, 211, 261) of the top plate (160, 210, 260) and a first side surface (181, 231, 281) of the bottom plate (180, 230, 280) toward a second side surface (163, 213, 263) of the top plate (160, 210, 260) and a second side surface (183, 233, 283) of the bottom plate (180, 230, 280).

9. The backstop device (150, 200, 250) of any of claims 1-8, wherein a volume of the top plate (160) is less than a volume of the bottom plate (180, 230, 280).

10. An injection device (101) comprising:
a syringe barrel (140);
a plunger rod (110) comprising an elongated body (102) extending along a central axis (104) between a proximal end (106) and a distal end (108), the body having a joining portion (114) joining a first curved end face (110) to a second curved end face (112), each of the first curved end face (110), the second curved end face (112), and the joining portion (114) extending between the proximal end (106) and the distal end (108), wherein the first curved end face (110) and the second curved end face (112) together define a discontinuous arcuate outer perimeter of the elongated body (102); and
the backstop device (150, 200, 250) according to any of claims 1-9.

11. The injection device (101) of claim 10, wherein a distance (W₁₆₆, W₂₁₆, W₂₆₆) between a first planar surface (167, 217, 267) and a second planar surface (169, 219, 269) of the first opening (166, 216, 266) of the top plate (160, 210, 260) is less than a diameter of the distal end (108) of the plunger rod (110).

12. The injection device (101) of claims 10 or 11, wherein the bottom plate (180, 230, 280) is separated from the top plate (160, 210, 260) by a distance such that, when the backstop device (150, 200, 250) is coupled to the syringe barrel (140), a lower surface (164) of the top plate (160, 210, 260) and an upper surface (182, 232, 282) of the bottom plate (180, 230, 280) contact an upper surface (157) and a lower surface (159) of a flange (158) of the syringe barrel (140), respectively.
